# EUROPÄISCHE PATENTSCHRIFT

(11) **EP 2 090 913 B1**
(45) Veröffentlichungstag und Bekanntmachung des Hinweises auf die Patenterteilung: **31.08.2016**
(21) Anmeldenummer: 09152585.7
(22) Anmeldetag: 11.02.2009
(51) Int. Cl.: G02B 7/24, G02B 21/00, G02B 7/00, G02B 21/20, G02B 21/22

(54) **Tubus für eine Beobachtungseinrichtung**
Tube for an observation device
Tubes pour un dispositif d'observation

(30) Priorität: 15.02.2008 DE 102008009303
(43) Veröffentlichungstag der Anmeldung: 19.08.2009
(73) Patentinhaber: Carl Zeiss Meditec AG, 07745 Jena (DE)
(72) Erfinder: Kolster, Nadine, 73447 Oberkochen (DE); Abele, Alfons, 73527, Schwäbisch Gmünd (DE); Lücke, Christian, 73447, Oberkochen (DE)
(74) Vertreter: Müller & Schubert

(56) Entgegenhaltungen:
- WO-A-98/38543
- DE-A1- 3 117 254
- US-A- 4 576 450
- US-A- 4 798 451
- US-A- 5 557 455

## Beschreibung

Die vorliegende Erfindung betrifft einen Tubus für eine Beobachtungseinrichtung gemäß dem Oberbegriff von Patentanspruch 1. Bei einer solchen Beobachtungseinrichtung kann es sich beispielsweise um ein Mikroskop oder dergleichen handeln.

Bei einem Mikroskop, beispielsweise einem Operationsmikroskop, wird der Abstand der Objektebene von der Lage der Geräte - Austrittspupille AP hinter dem Okular, also die Einblickhöhe, durch die von der jeweiligen Anwendung abhängigen Gerätekonfiguration der Systemkomponenten des Mikroskops bestimmt.

Aus ergonomischen Gründen ist natürlich eine individuelle Anpassung der Einblickhöhe an die Körpergröße des Mikroskopbenutzers, beispielsweise eines Operateurs, äußerst vorteilhaft und daher wünschenswert. Bei den oft zeitaufwendigen Arbeiten mit dem Operationsmikroskop ist eine entspannte Körperhaltung des Operateurs ein ganz entscheidender Faktor für ein ermüdungsfreies Arbeiten.

Weiterhin ist entscheidend, dass der vom Operateur gewohnte Standard für die optischen Grundgrößen, wie beispielsweise die Gesamtvergrößerung, der Objektfelddurchmesser, die Objektauflösung, der freie Arbeitsabstand und die optische Abbildungsqualität bei einer Änderung der Einblickhöhe nicht nachteilig verändert wird.

Im Hinblick auf Tuben mit veränderlichen Einblickhöhen sind im Stand der Technik bereits eine ganze Reihe unterschiedlicher Lösungen bekannt geworden.

Ein Lösungsvorschlag beinhaltet beispielsweise eine rein mechanische Abstandsänderung zwischen dem Mikroskopkörper und dem Tubus.

Eine andere Lösung ist beispielsweise in der DE 34 15 958 A1 beschrieben. Dort ist ein Tubus mit variabler Einblickhöhe für optische Geräte offenbart, wobei der Tubus in zwei unterschiedliche Positionen gedreht werden kann. Allerdings sind nur zwei Einstellmöglichkeiten möglich, in die der Tubus eingeschwenkt und eingerastet werden kann. Eine lineare Verschiebung oder Variation des Tubus ist nicht vorgesehen.

Eine weitere Lösung ergibt sich beispielsweise aus der EP 1 233 294 B1. In dieser Patentschrift wird eine umschaltbare, also stufenweise Änderung der Einblickhöhe mittels einer Tubuskonstruktion mit variabler Baulänge erreicht. Die mechanische Variation der Baulänge erfolgt dabei durch einen teleskopisch ausziehbaren Bereich im Tubus. Für die dafür notwendige Veränderung der optischen Tubuslänge durch Verschiebung der Fokuslage des Zwischenbilds weist der Tubus im konvergenten optischen Strahlengang einen veränderbaren Bereich auf. In diesem veränderbaren Bereich werden zur Veränderung der optischen Tubuslänge in einem ersten Lösungsvorschlag ein- und ausschiebbare und in einem zweiten Lösungsvorschlag ein- und ausschwenkbare Korrekturlinsen oder - linsengruppen eingesetzt. Diese Korrekturlinsen haben positive oder negative Brechkraft und verschieben das von der Tubusoptik erzeugte Zwischenbild in das feststehende Sehfeld des Okulars, wobei der Durchmesser des Zwischenbilds, in mm ausgedrückt durch die Sehfeldzahl SFZ, durch eine im Okular angebrachte Sehfeldblende begrenzt wird. Dadurch wird im Okular "etwa derselbe".Bildausschnitt in der "gewünschten" Vergrößerung sichtbar und scharf abgebildet.

Bei den geschilderten Lösungsvorschlägen wird dann durch mechanische Abstandsänderung in einem teleskopisch ausziehbaren Bereich das vom Tubus erzeugte und mit den Zusatzoptiken verschobene Zwischenbild mit dem Sehfeld des Okulars zur Deckung gebracht.

Bei der rein mechanischen Verlängerung des afokalen Strahlengangs zwischen den Komponenten muss mit zunehmender Abstandsänderung auch mit zunehmender Vignettierung, also Helligkeitsabfall von der Bildfeldmitte zum Bildfeldrand oder sogar mit Bildfeldbeschnitt am Bildfeldrand gerechnet werden. Zwar werden bei diesem Lösungsansatz die optischen Grundgrößen nicht verändert, man muss aber durch die Vignettierung doch eine mehr oder weniger starke Minderung der Abbildungsqualität in Kauf nehmen.

Als wesentlicher Nachteil der EP 1 233 294 B1 muss der hohe mechanische Aufwand für den optischen Korrekturmechanismus aus Schwenk- beziehungsweise Schiebelinsen angesehen werden. Problematisch ist auch der dafür benötigte Platzbedarf, was zu einem deutlich höheren Bauvolumens führt.

Bei einer weiteren Tubusart handelt es sich um die so genannten Schwenktuben. Schwenktuben an sich sind bereits aus dem Stand der Technik bekannt. Aus der DE 197 07 520 A1 beispielsweise ist ein Schwenktubus für ein optisches Beobachtungsgerät, etwa ein Mikroskop, bekannt. Der Schwenktubus verfügt über ein Basisteil sowie einen Okularträger, wobei die Schwenkbewegung dadurch realisiert wird, dass sich der Okularträger auf dem Basisteil längs eines Kreisbogens abwälzt. Im Basisteil ist ein um eine Drehachse drehbar gelagerter Umlenkspiegel vorgesehen, wobei dieser Umlenkspiegel durch die Abwälzbewegung des Okularträgers auf dem Basisteil zwangsgedreht wird. Ebenso ist auch im Okularträger ein um eine Drehachse drehbar gelagerter Umlenkspiegel vorgesehen, wobei auch dieser Umlenkspiegel durch die Abwälzbewegung des Okularträgers auf dem Basisteil zwangsgedreht wird.

Bei dieser bekannten Lösung ist die Verschwenkung der Spiegel stets an die Abwälzbewegung gekoppelt. Dadurch bewegen sich die beiden Spiegel beim Schwenken jeweils um den gleichen Winkel sowie miteinander gekoppelt. Die Austrittspupille kann mit der bekannten Lösung in einem Schwenkbereich von ±60° verschwenkt werden. Dieser realisierbare Schwenkbereich ist jedoch für viele Anwendungsfälle nicht ausreichend.

In der DE 103 16 242 A1 ist ein Schwenktubus beschrieben, der um 180° verschwenkt werden kann und deshalb 180°-Schwenktubus genannt wird. Dazu weist der Schwenktubus ein erstes Optikelement in Form eines Prismas auf, das sich aus einer waagerechten Position um jeweils 45° nach oben und unten auslenken lässt. Relativ dazu lässt sich in einer zweiten Achse ein weiteres Prisma gleichzeitig auch um jeweils 45° bewegen. Zusammen ergibt sich daraus dann der geforderte Winkel von ±90°.

In manchen bekannten Lösungen baut der Schwenktubus bei geradem Einblick, insbesondere bei bestimmten Anwendungen (zum Beispiel in der Neurochirurgie), zu lang und bei so genannten Face-To-Face-Anwendungen mit einem Einblickwinkel (das ist der abweichende Winkel der Orthogonalen zur Mikroskopachse) von 15° bis 30° zu kurz. Dies liegt unter anderem daran, dass der Abstand zur Mikroskopachse zu gering ist. Letzteres trifft insbesondere bei Anwendungen in der Ophthalmoskopie zu.

In der US 4,576,450 ist ein Mikroskoptubus beschrieben, welcher schwenkbar ausgebildet ist. Dabei kann die Einblickhöhe des Tubus i9n Höhe und/oder Länge verändert werden. Im optischen Lichtweg des Tubus sind optische Elemente in Form von Umlenkspiegeln vorgesehen, die unabhängig voneinander bewegt werden können. Die Umlenkspiegel liegen im bereich der drehgelenke und sind mit diesen gekoppelt.

Ausgehend vom genannten Stand der Technik liegt der vorliegenden Erfindung die Aufgabe zugrunde, einen Tubus für eine Beobachtungseinrichtung, insbesondere für ein Mikroskop, bereitzustellen, mittels dessen für jeden Einblickwinkel unterschiedliche Einblickpositionen beziehungsweise Baulängen und Bauhöhen des Tubus realisierbar sind.

Diese Aufgabe wird erfindungsgemäß gelöst durch den Tubus mit den Merkmalen gemäß dem unabhängigen Patentanspruch 1. Weitere Merkmale, Details, Aspekte, Vorteile und Effekte der Erfindung ergeben sich aus den Unteransprüchen, der Beschreibung sowie den Zeichnungen.

Erfindungsgemäß wird ein Tubus, insbesondere ein Schwenktubus, für eine optische Beobachtungseinrichtung, insbesondere für ein Mikroskop, bereitgestellt, mit einer in Länge und/oder Höhe linear variablen Einblickhöhe, wobei der Tubus einen optischen Lichtweg aufweist, in dem wenigstens zwei optische Umlenkelemente angeordnet sind, wobei wenigstens zwei optische Umlenkelemente unabhängig voneinander beweglich im optischen Lichtweg angeordnet sind, wobei der Tubus ein Basisteil zur Anlage an einer optischen Beobachtungseinrichtung aufweist, in dem ein um eine erste Drehachse drehbares Umlenkelement angeordnet ist, wobei der Tubus einen zum Basisteil verschwenkbaren Okularträger aufweist, in dem ein um eine zweite Drehachse drehbares Umlenkelement angeordnet ist, wobei der Okularträger über wenigstens einen Zwischenträger verschwenkbar am Basisteil angeordnet ist, wobei der Zwischenträger über ein Drehgelenk mit dem Basisteil und über ein Drehgelenk mit dem Okularträger verbunden ist, wobei das Umlenkelement des Basisteils mit dem Drehgelenk zwischen Basisteil und Zwischenträger gekoppelt ist und wobei dass das Umlenkelement des Okularträgers mit dem Drehgelenk zwischen Okularträger und Zwischenträger gekoppelt ist. Der Tubus ist dadurch gekennzeichnet, dass das Umlenkelement des Basisteils mit einem Koppelelement verbunden ist, welches mit einem Mitnehmerelement, welches mit dem Basisteil verbunden ist, und mit einem Mitnehmerelement, welches mit dem Zwischenträger verbunden ist, in Verbindung steht, und dass das Umlenkelement des Okularträgers mit einem Koppelelement verbunden ist, welches mit einem Mitnehmerelement, welches mit dem Okularträger verbunden ist, und mit einem Mitnehmerelement, welches mit dem Zwischenträger verbunden ist, in Verbindung steht.

Gemäß der vorliegenden Erfindung sollen wenigstens zwei optische Elemente vorgesehen sein, wobei die Erfindung nicht auf eine bestimmte Anzahl beschränkt ist. Je nach Ausgestaltung können auch mehr als zwei solcher optischer Elemente vorgesehen sein. Ebenso ist die Erfindung nicht auf eine bestimmte Ausgestaltung der optischen Elemente beschränkt. Einige vorteilhafte, jedoch nicht ausschließliche Beispiele hierzu werden im weiteren Verlauf der Beschreibung näher erläutert.

Erfindungsgemäß ist vorgesehen, dass wenigstens zwei optische Elemente unabhängig voneinander, das heiß individuell, beweglich sein sollen. Das schließt den Fall ein, dass alle optischen Elemente derart beweglich sind. Es ist aber auch der Fall eingeschlossen, dass mehr als zwei optische Elemente vorgesehen sind und dass dann wenigstens eines der optischen Elemente nicht in der genannten Weise beweglich ist.

Ebenso ist die Erfindung nicht darauf beschränkt, wie die einzelnen optischen Elemente bewegt werden können/sollen. Beispielsweise können diese gedreht und/oder verschoben werden. Auch sind beliebige Kombinationen daraus denkbar, etwa das wenigstens ein optisches Element verdreht und wenigstens eines verschoben wird. Zusätzlich kann weiterhin auch noch wenigstens ein optisches Element vorgesehen sein, das nicht beweglich ist.

In vorteilhafter Ausgestaltung ist vorgesehen, dass mindestens zwei optische Elemente vorgesehen sind, die unabhängig voneinander verschiebbar und/oder verdrehbar sind.

Vorteilhaft kann vorgesehen sein, dass der Tubus derart ausgebildet ist, dass sich der optische Lichtweg (hierbei handelt es sich um die optische Weglänge des Tubus) des Tubus bei einer Variierung der Einblickhöhe, etwa bei einer Längenänderung des Tubus, um weniger als 30% ändert. Vorteilhaft ändert sich der optische Lichtweg um weniger als 10%, weiter bevorzugt um weniger als 5%. Ganz besonders bevorzugt ist es, wenn sich der optische Lichtweg gar nicht oder nur geringfügig ändert, das heißt, wenn der optische Lichtweg konstant oder aber zumindest annähernd konstant ist beziehungsweise bleibt.

Ein erfindungsgemäßer Tubus könnte sich daher auch wie folgt darstellen, nämlich mit wenigstens einem durch den Tubus hindurch tretenden Strahlengang, wobei in dem Strahlengang wenigstens zwei optische Elemente vorgesehen sind, wobei der Tubus einen konstanten oder annähernd konstanten optischen Lichtweg aufweist, in dem sich die optischen Elemente befinden, und wobei wenigstens zwei optische Elemente unabhängig voneinander beweglich angeordnet sind.

Vorteilhaft ist ein Tubus für eine optische Beobachtungseinrichtung, insbesondere für ein Mikroskop, mit einer in Länge und/oder Höhe linear variablen Einblickhöhe, wobei der Tubus einen optischen Lichtweg aufweist, in dem wenigstens zwei optische Elemente angeordnet sind, insbesondere ein wie vorstehend beschriebener erfindungsgemäßer Tubus, der dadurch gekennzeichnet ist, dass der Tubus in seiner mechanischen Länge variabel einstellbar ausgebildet ist. Unter der mechanischen Länge ist dabei die physikalische, das heißt die körperliche Länge des Tubus zu verstehen. Sie entspricht damit der tatsächlich linearen Ausdehnung des Tubus. Die mechanische Länge muss nicht zwangsläufig mit der optischen Weglänge (dem optischen Lichtweg) identisch sein. Bevorzugt ist gemäß der vorliegenden Erfindung, wenn sich bei einer mechanischen Längenänderung des Tubus dessen optische Weglänge (dessen optischer Lichtweg) nicht, oder aber nur geringfügig ändert.

Der Vorteil eines verstellbaren Tubus, bei dem sich die optische Weglänge bei dessen mechanischer Längenänderung (etwa beim "Ausziehen") nicht verändert, liegt darin, dass sich bei Verstellen des Tubus dann die Vergrößerung des Systems nicht ändert und dass die Lage der Austrittspupille konstant bleibt.

Ein optischer Tubus für eine Beobachtungsvorrichtung, insbesondere für ein Operationsmikroskop, kann dadurch gekennzeichnet sein, dass seine mechanische Länge (das heißt die Entfernung vom Okular zur Verbindungsstelle mit dem Rest der Beobachtungsvorrichtung) variabel einstellbar ist, wobei alle wesentlichen optischen Komponenten fest in dem Strahlengang installiert sind oder sein können. Das heißt, das keine optischen Komponenten vorhanden sind, die eingeschwenkt oder umgebaut werden müssen, um eine Längenänderung bei Beibehaltung anderer optischer Eigenschaften zu bewirken.

Vorzugsweise kann vorgesehen sein, dass die Längenänderung mindestens 10%, bevorzugt 30%, weiter bevorzugt 50%, besonders bevorzugt mindestens 100% betragen kann.

Vorteilhaft kann die Anzahl der Änderungsabstufungen größer als 2, bevorzugt größer als 5 weiter bevorzugt größer als 20, ganz besonders bevorzugt unendlich sein.

In weiterer Ausgestaltung kann vorgesehen sein, dass der optische Strahlengang gefaltet ist.

Wie weiter oben schon ausgeführt wurde, können die optischen Elemente auf unterschiedliche Weise ausgebildet sein. Beispielsweise kann es sich um Linsen, Linsensysteme, Linsengruppen, Prismen oder dergleichen handeln. Erfindungsgemäß sind die optischen Elemente als Umlenkelemente ausgebildet. Die vorliegende Erfindung ist nicht auf bestimmte Typen von Umlenkelementen beschränkt. Vorteilhaft sind die Umlenkelemente jedoch als Umlenkspiegel ausgebildet. Bei den Umlenkelementen könnte es sich aber generell auch um entsprechend ausgestaltete Umlenkprismen oder dergleichen handeln. Es sind natürlich auch Kombinationen unterschiedlicher optischer Elemente denkbar.

Vorteilhaft kann vorgesehen sein, dass der Tubus linear verschiebbar ausgebildet ist. Beispielsweise kann dann vorgesehen sein, dass ein optisches Element parallel zur Verbindungslinie zwischen dem anderen optischen Element und dem Anschluss zur Beobachtungseinrichtung verschiebbar angeordnet ist. Vorzugsweise kann die Verschiebbarkeit auf einer Geraden liegen beziehungsweise erfolgen Dann kann wenigstens ein optisches Element (vorzugsweise zwei optische Elemente) auf einer einzigen Geraden verschiebbar angeordnet sein. Durch eine Bewegung des optischen Elements kann dann der Fokus angepasst werden.

Grundsätzlich kann der Tubus in einer Weise ausgebildet sein, dass er ein Basiselement zur Anlage an die optische Beobachtungseinrichtung aufweist. Über das Basiselement kann dann der Tubus an der Beobachtungseinrichtung angeordnet beziehungsweise befestigt werden.

Die vorliegende Erfindung ist nicht auf bestimmte Typen von Beobachtungseinrichtungen beschränkt ist. Bei der Beobachtungseinrichtung kann es sich beispielsweise um ein Mikroskop, etwa ein Operationsmikroskop, oder dergleichen handeln.

Beispielsweise kann der Tubus für einen monokularen Einblick (ein Strahlengang), oder aber auch als Binokulartubus (zwei getrennte Strahlengänge für Stereomikroskopie) ausgestaltet sein. Im letztgenannten Fall können die optischen Elemente vorzugsweise von beiden Strahlengängen gemeinsam genutzt werden.

In vorteilhafter Ausgestaltung kann der Tubus als Schwenktubus ausgebildet sein. In einer einfachen Ausführung kann eine zweigelenkige Ausgestaltung vorgesehen sein, bei der ein Okularträger schwenkbeweglich an dem Basisteil befestigt ist. Es sind jedoch auch dreigelenkige Verbindungen denkbar, bei denen der Okularträger schwenkbeweglich an einem Zwischenträger und über diesen schwenkbeweglich am Basiselement angeordnet ist. Bei einer viergelenkigen Ausgestaltung ist der Okularträger in entsprechender Weise über zwei Zwischenträger, die untereinander schwenkbeweglich verbunden sind, mit dem Basisteil verbunden.

Erfindungsgemäß wird ein Schwenktubus für eine Beobachtungseinrichtung, insbesondere für ein Mikroskop, bereitgestellt, mit einem Basisteil, wobei in dem Basisteil ein um eine erste Drehachse drehbares Umlenkelement angeordnet ist, und mit einem zum Basisteil verschwenkbaren Okularträger, wobei in dem Okularträger ein um eine zweite Drehachse drehbares Umlenkelement angeordnet ist. Der Schwenktubus ist dadurch gekennzeichnet, dass die Umlenkelemente unabhängig voneinander drehbar und/oder verschiebbar angeordnet sind.

Ein solcher Schwenktubus besteht zunächst aus einem Basisteil sowie einem dazu verschwenkbaren Okularträger. Die Verschwenkung kann stattfinden, indem der Okularträger um das Basisteil verschwenkt wird, oder indem das Basisteil um den Okularträger verschenkt wird, oder indem Basisteil und Okularträger beide umeinander verschwenkt werden.

Das Basisteil verfügt über ein Umlenkelement zum Umlenken der Strahlung, das um eine Drehachse drehbar im Basisteil angeordnet ist. Der Okularträger, der zum Tragen des Okulars beziehungsweise der dafür erforderlichen optischen Elemente dient, verfügt ebenfalls über ein Umlenkelement zum Umlenken der Strahlung, das um eine Drehachse drehbar im Okularträger angeordnet ist.

Vorteilhaft ist vorgesehen, dass beide Umlenkelemente unabhängig voneinander gedreht und/oder verschoben werden können. Damit wird insbesondere ein ergonomischer Schwenktubus geschaffen, der multidisziplinär an einer Beobachtungseinrichtung, beispielsweise an einem Mikroskop, eingesetzt werden kann. Durch den Einsatz von zwei unabhängig voneinander drehbaren beziehungsweise schwenkbaren und/oder verschiebbaren Umlenkelementen können für nahezu jeden Einblickwinkel unterschiedliche, ergonomische Einblickpositionen beziehungsweise Bauhöhen und Baulängen des Schwenktubus realisiert werden.

Dabei ist in vorteilhafter Ausgestaltung bei geradem Einblick (Schwenkwinkel 0°) eine Variation der Baulänge von beispielsweise ±30 mm möglich.

Ebenso können Einblickwinkel von größer 90°, beispielsweise bis 100°, realisiert werden.

Dies wird insbesondere dadurch erreicht, dass die Dreh- beziehungsweise Schwenkbewegung der beiden Umlenkelementen nicht mehr direkt aneinander gekoppelt ist, wie dies beispielsweise bei der in der DE 197 07 520 A1 beschriebenen Lösung der Fall ist.

Vorteilhaft können die optischen Umlenkelemente, eine translatorische Komponente enthalten.

In weiterer Ausgestaltung können die Umlenkelemente auch rotatorisch unabhängig voneinander schwenkbar angeordnet sein. Das bedeutet, dass die beiden Umlenkelemente völlig unabhängig voneinander gedreht beziehungsweise verschwenkt und verschoben werden können, wobei neben der Bewegung auf einem Kreisbogen auch noch eine lineare Komponente der Bewegung existiert.

Erfindungsgemäß ist der Okularträger über wenigstens einen Zwischenträger verschwenkbar am Basisteil angeordnet. Dabei ist die Erfindung nicht auf bestimmte Ausführungsformen des Zwischenträgers beschränkt. Dieser weist vorzugsweise eine langgestreckte Form auf, wobei "langgestreckt" bedeutet, dass der Zwischenträger eine größere Länge als Breite aufweist. Ebenso ist die Erfindung nicht auf eine bestimmte Anzahl von Zwischenträgern beschränkt. Beispielsweise können auch zwei oder mehr Zwischenträger vorgesehen sein.

Erfindungsgemäß ist der Zwischenträger über ein Drehgelenk mit dem Basisteil verbunden. Zusätzlich ist der Zwischenträger über ein Drehgelenk auch mit dem Okularträger verbunden. Dabei ist die Erfindung nicht auf bestimmte Typen von Drehgelenken beschränkt. Wenn zwei oder mehr Zwischenträger vorgesehen sind, können auch diese über ein Drehgelenk miteinander verbunden sein.

Vorteilhaft kann/können das Umlenkelement des Basisteils und/oder das Umlenkelement des Okularträgers im Bereich eines Drehgelenks angeordnet sein.

Insbesondere von Vorteil ist es, wenn hier eine entsprechende Kopplung realisiert wird. So ist erfindungsgemäß vorgesehen, dass das Umlenkelement des Basisteils mit dem Drehgelenk zwischen Basisteil und Zwischenträger gekoppelt ist. Zusätzlich ist das Umlenkelement des Okularträgers mit dem Drehgelenk zwischen Okularträger und Zwischenträger gekoppelt. Die Kopplung kann auf unterschiedlichste Weise erfolgen, so dass die Erfindung nicht auf bestimmte Kopplungsmechanismen beschränkt ist. Erfindungsgemäß ist das Umlenkelement mit einem Koppelelement verbunden, wobei das Koppelelement wiederum mit einem Mitnehmerelement zusammenwirkt, welches an dem Drehlager angeordnet ist.

Vorteilhaft können das Umlenkelement des Basisteils und das entsprechende Drehgelenk eine identische Drehachse aufweisen. Alternativ oder zusätzlich können auch das Umlenkelement des Okularträgers und das entsprechende Drehgelenk eine identische Drehachse aufweisen.

Bezüglich der Rotationswinkel von Umlenkelement und Drehgelenk kann vorteilhaft vorgesehen sein, dass das Umlenkelement des Basisteils um einen Rotationswinkel um die Drehachse rotierbar angeordnet ist, der der Hälfte des Rotationswinkels des Drehgelenks um die Drehachse entspricht.

In weiterer Ausgestaltung kann vorgesehen sein, dass das Umlenkelement des Okularträgers um einen Rotationswinkel um die Drehachse rotierbar angeordnet ist, der der Hälfte des Rotationswinkels des Drehgelenks um die Drehachse entspricht.

Vorteilhaft kann vorgesehen sein, dass die Drehachse des ersten Umlenkelements parallel zur Drehachse des zweiten Umlenkelements angeordnet ist.

In weiterer Ausgestaltung kann vorgesehen sein, dass in dem Basisteil wenigstens ein optisches Auskoppelelement und/oder wenigstens ein optisches Einkoppelelement angeordnet ist/sind. Dabei kann es sich vorteilhaft um Teilerelemente handeln, wie beispielsweise Teilerwürfel, Teilerspiegel und dergleichen. Diese Elemente können beispielsweise im Zusammenhang mit Dokumentationseinrichtungen, Dateneinspiegelungseinrichtungen und dergleichen einsetzbar sein. Dabei kann vorteilhaft vorgesehen sein, dass das/die Element(e) anstelle einer ansonsten oftmals eingesetzten Glasplatte verwendet werden.

Vorteilhaft können oberhalb einer Tubusanlagefläche (zur Anlage an der optischen Beobachtungseinrichtung) in dessen Basisteil optische Teiler zu den oben genannten Zwecken vorgesehen sein, was beispielsweise die Einsparung zusätzlicher optischer Bauteile ermöglicht. Eine Auskopplungsrichtung kann beispielsweise auch seitlich oder nach hinten auf eine vom Beobachter abgewandte Seite erfolgen.

Durch die vorliegende Erfindung wird ein ergonomischer Tubus, beispielsweise ein Schwenktubus, mit variabler Einblickrichtung und variabler Lage der Austrittspupille geschaffen.

Vorteilhaft kann es sich bei der Austrittspupille um eine Blende handeln, beziehungsweise die Austrittspupille als Blende ausgebildet sein. In weiterer Ausgestaltung kann vorgesehen sein, dass die Austrittspupille des Okulars für verschiedene Einblickwinkel und/oder Einblickpositionen vorteilhaft in einem Lagebereich LAP liegt.

Im Falle eines Schwenktubus kann vorgesehen sein, dass durch eine Kopplung der Umlenkelementen von Basisteil mit Zwischenträger beziehungsweise Zwischenträger mit Okularträger die beiden Drehachsen (Spiegelachsen, wenn die Umlenkelemente Umlenkspiegel sind) unabhängig voneinander beweglich sind. Dadurch kann die Austrittspupille im Lagebereich frei bewegt werden. Auch lineare Bewegungen bei gleichem Schwenkwinkel sind möglich. Es gibt keine Kopplung der beiden Drehachsen der Umlenkelemente. Im Unterschied dazu ist in der DE 197 07 520 A1 ein Schwenktubus mit variabler Einblickrichtung beschrieben, wobei der Okularträger durch eine Abwälzbewegung mittels kämmender Zahnräder auf dem Basisteil zwangsgedreht wird. Durch die Zwangskopplung zwischen Basisteil und Okularträger bewegen sich die beiden Umlenkelemente beim Schwenken gekoppelt und um den gleichen Winkel.

Insbesondere wird es durch den erfindungsgemäßen Tubus, beispielsweise einen erfindungsgemäßen Schwenktubus, auch möglich, bis auf die Endanschläge für jeden Einblickwinkel unterschiedliche Einblickpositionen und/oder Bauhöhen und/oder Baulängen realisieren zu können.

Zusammenfassend kann die vorliegende Erfindung auch wie folgt beschrieben werden. Es wird ein Tubus für eine optische Beobachtungseinrichtung, insbesondere für ein Mikroskop, beschrieben, mit einer in Länge und/oder Höhe linear variablen Einblickhöhe, wobei der Tubus einen optischen Lichtweg aufweist, in dem wenigstens zwei optische Umlenkelemente angeordnet sind. Der Tubus kann beispielsweise als Schwenktubus ausgebildet sein, mit einem Basisteil, wobei in dem Basisteil ein um eine Drehachse drehbares Umlenkelement angeordnet ist, und mit einem zum Basisteil verschwenkbaren Okularträger, wobei in dem Okularträger ein um eine Drehachse drehbares Umlenkelement angeordnet ist. Um einen großen Schwenkbereich zu ermöglichen, bei dem gleichzeitig für jeden Einblickwinkel unterschiedliche Einblickpositionen beziehungsweise Baulängen und Bauhöhen des Schwenktubus realisierbar sind, wird erfindungsgemäß vorgeschlagen, dass die optischen Elemente unabhängig voneinander beweglich im optischen Lichtweg angeordnet sind. Beispielsweise können Umlenkelemente in einem Schwenktubus unabhängig voneinander drehbar und/oder verschiebbar angeordnet sein.

Die Erfindung wird nun anhand von Ausführungsbeispielen unter Bezugnahme auf die beiliegenden Zeichnungen näher erläutert. Es zeigen
- Figuren 1 bis 5: in schematischer Ansicht unterschiedliche Einblickwinkel und Einblickpositionen, die mit dem erfindungsgemäßen Tubus realisiert werden können;
- Figuren 6 bis 11: ein Ausführungsbeispiel eines erfindungsgemäßen Tubus in verschiedenen Einblickpositionen;
- Figuren 12 und 13: verschiedene Winkelzusammenhänge beim Verschwenken eines erfindungsgemäßen Tubus;
- Figur 14: in schematischer Ansicht ein anderes Ausführungsbeispiel eines erfindungsgemäßen Tubus in verschiedenen Einblickpositionen; und
- Figuren 15 bis 18: in schematischer Ansicht noch ein anderes Ausführungsbeispiel eines erfindungsgemäßen Tubus.

In den Figuren ist ein Tubus dargestellt, der als Schwenktubus 10 ausgebildet ist, und der für eine optische Beobachtungseinrichtung, beispielsweise ein Mikroskop, etwa ein Operationsmikroskop, verwendet werden kann.

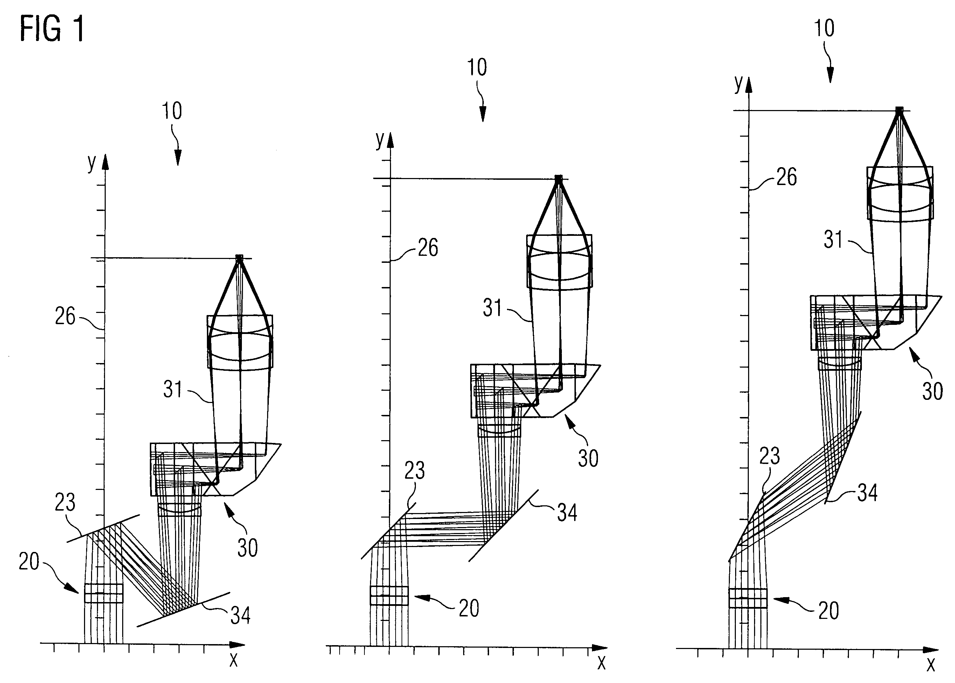

Der in den Figuren 1 bis 5 dargestellte Schwenktubus 10 verfügt über ein Basisteil 20, in dem ein um eine Drehachse drehbar angeordnetes Umlenkelement 23 in Form eines Umlenkspiegels vorgesehen ist. Weiterhin ist ein Okularträger 30 mit einer Okularaufnahme 31 vorgesehen, wobei auch der Okularträger 30 ein um eine Drehachse drehbar angeordnetes Umlenkelement 34 in Form eines Umlenkspiegels aufweist. Die beiden Umlenkspiegel 23, 34 sind sowohl translatorisch als auch rotatorisch unabhängig voneinander verschiebbar beziehungsweise drehbar angeordnet.

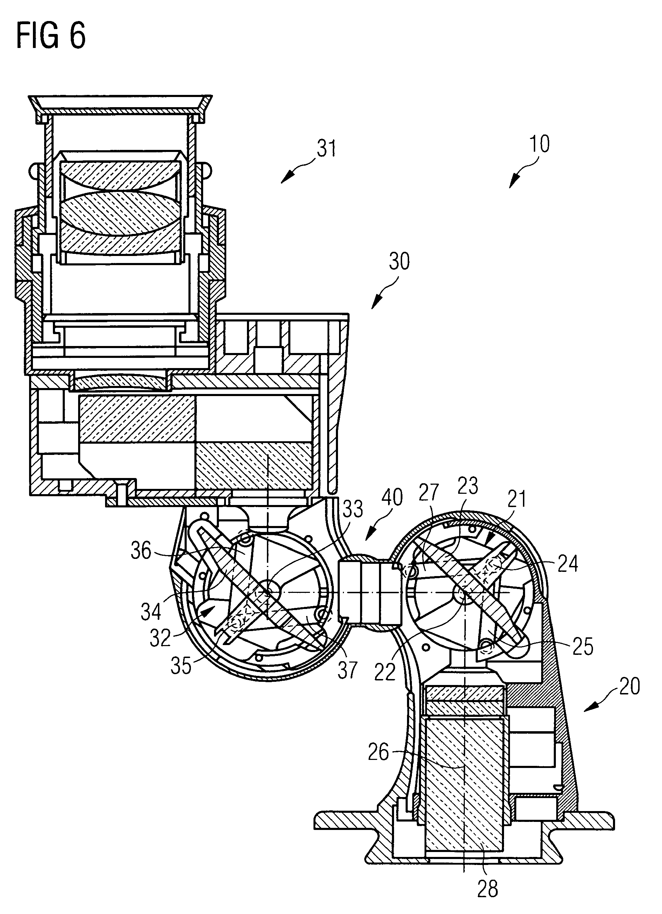

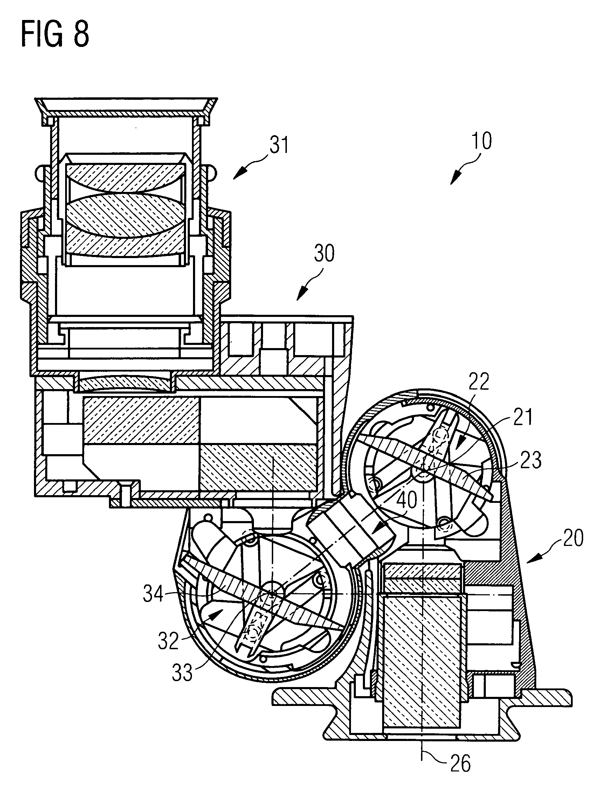

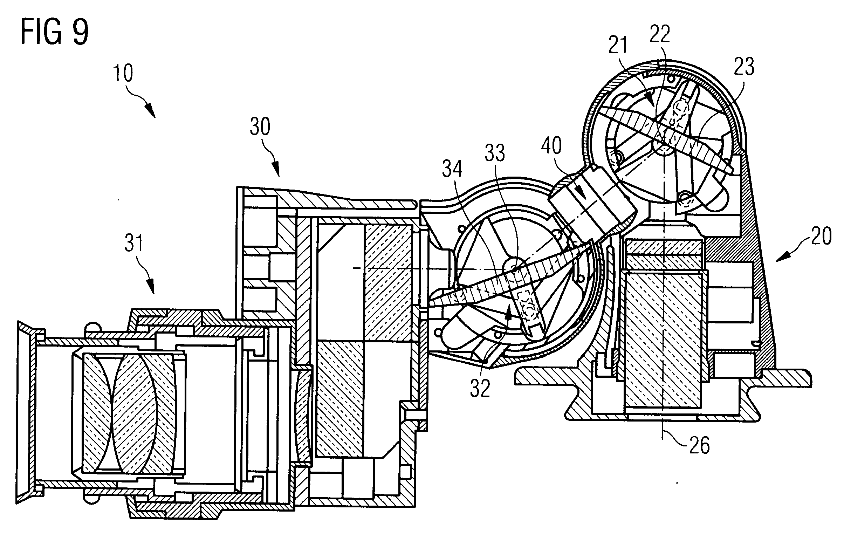

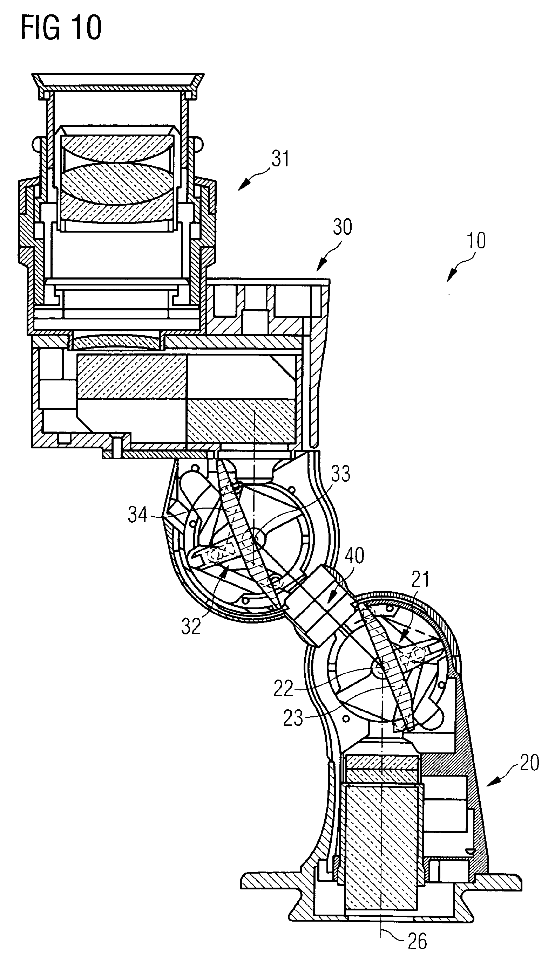

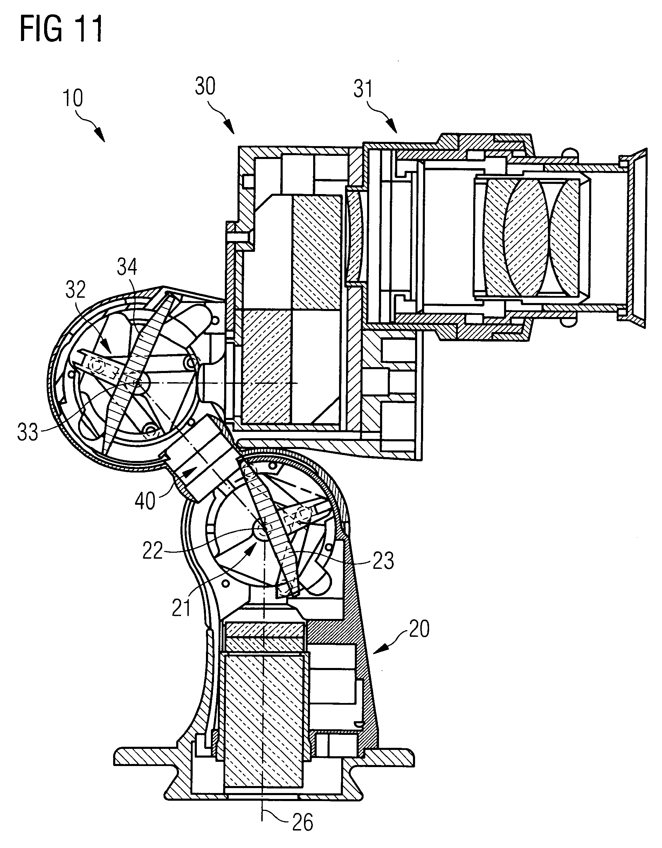

Das Verdrehen der Umlenkspiegel 23, 34 erfolgt um die entsprechenden Drehachsen, die im Zusammenhang mit den Figuren 6 bis 11 in größerem Detail dargestellt sind. Die translatorische Verschiebung erfolgt gemäß den Figuren 1 bis 5 innerhalb eines X-Y-Koordinatensystems, wobei die Y-Koordinate auch als optische Achse 26 des Basisteils 20 ausgebildet sein kann.

Wie in den Figuren 1 bis 5 dargestellt ist, können durch den Einsatz der beiden unabhängig voneinander schwenkbaren Umlenkspiegel 23, 34 für nahezu jeden Einblickwinkel unterschiedliche, ergonomische Einblickpositionen realisiert werden.

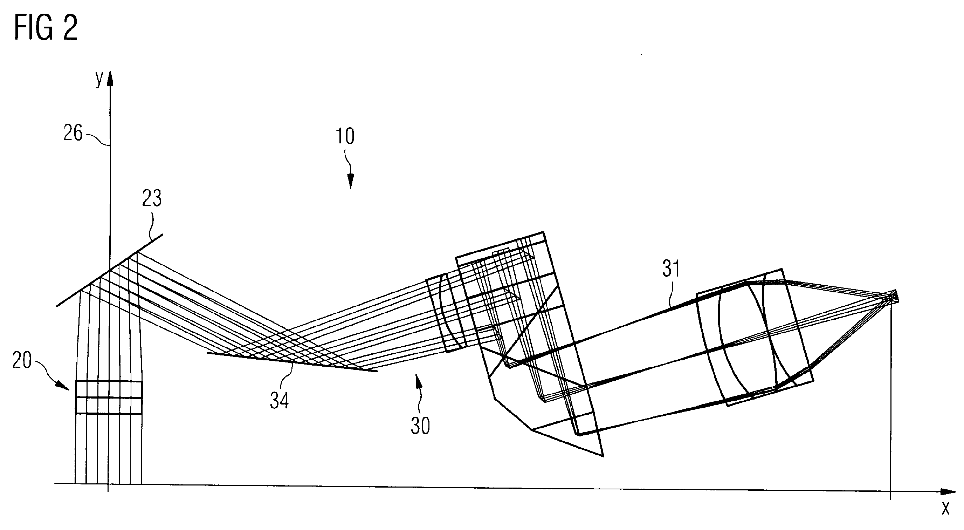

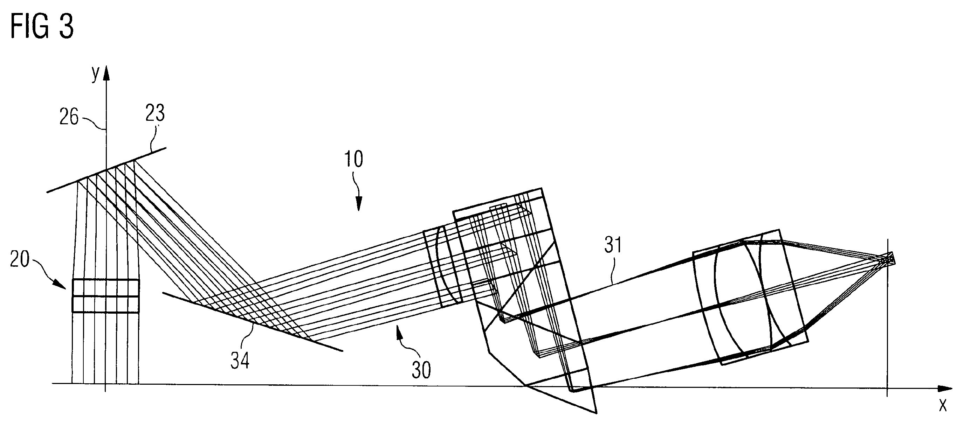

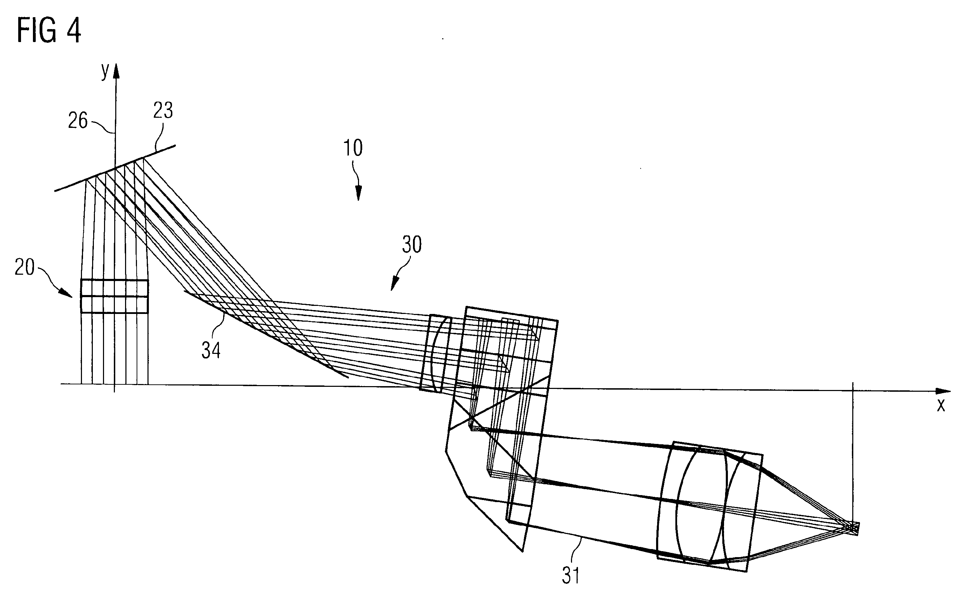

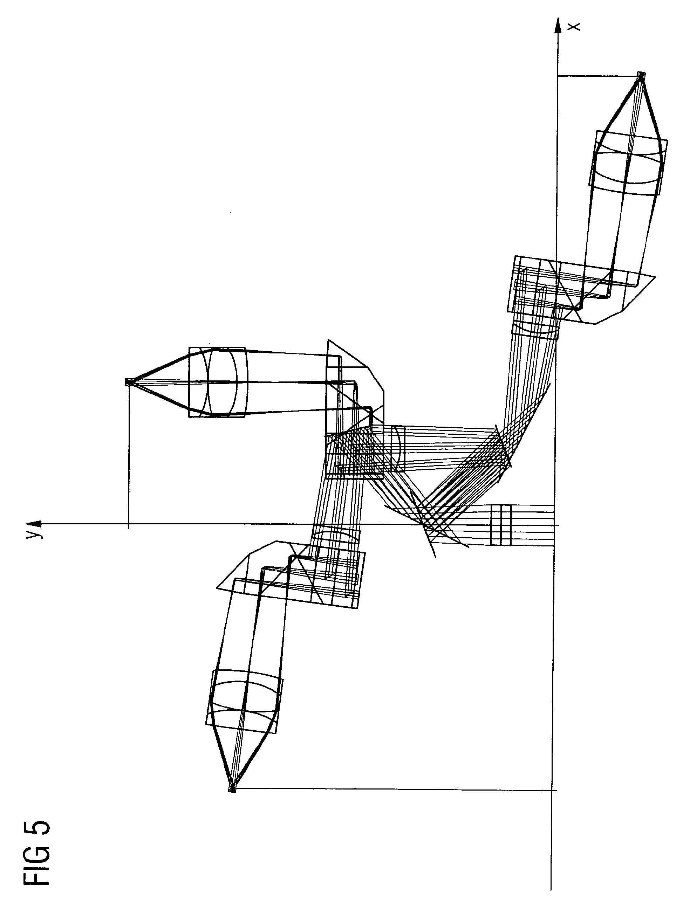

In der Figur 1 sind für den geraden Einblick drei unterschiedliche Baulängen dargestellt. Dabei kann die Baulänge beispielsweise um ±30 mm variiert werden. Die Figuren 2 und 3 zeigen einen schrägen Einblick mit einem Einblickwinkel von 15° (dies entspricht einem Schwenkwinkel von 75°). Figur 2 zeigt eine Spiegelkippung gemäß einer Variante 1, während Figur 3 eine Spiegelkippung gemäß einer Variante 2 darstellt. Im Vergleich zur Variante 1 ist bei Variante 2 der Abstand zur Achse 26 der Beobachtungseinrichtung um etwa 10 mm geringer. Die Einblickhöhe ist gegenüber der Variante 1 um etwa 15 mm niedriger. Figur 4 zeigt eine Verschwenkposition mit einem Einblickwinkel von -8° (das entspricht einem Schwenkwinkel von 98°). In Figur 5 schließlich ist ein kompletter Schwenkbereich des Schwenktubus 10 dargestellt, wobei dieser komplette Schwenkbereich größer 180° ist.

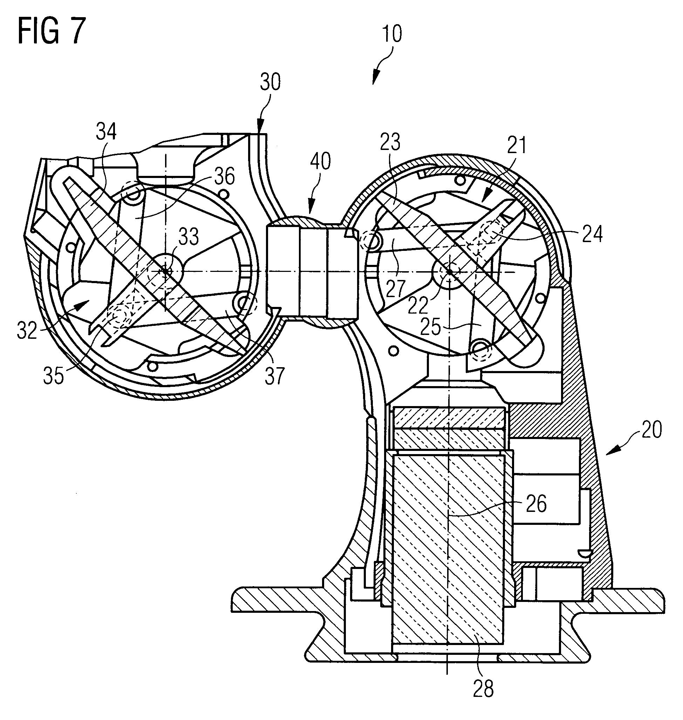

Die Figuren 6 bis 11 zeigen ein Ausführungsbeispiel eines erfindungsgemäßen Schwenktubus 10, wobei der Schwenktubus 10 in den einzelnen Figuren in verschieden Einblickpositionen dargestellt ist. Figur 7 stellt dabei einen vergrößerten Ausschnitt des in Figur 6 gezeigten Schwenktubus 10 dar.

Der Schwenktubus 10 verfügt zunächst über ein Basisteil 20, das über ein Drehgelenk 21 mit einem Zwischenträger 40 verbunden ist. Der Zwischenträger 40 ist weiterhin über ein Drehgelenk 32 mit einem Okularträger 30 verbunden, der eine Okularaufnahme 31 trägt.

Der Basisteil 20 verfügt weiterhin über ein Umlenkelement 23 in Form eines Umlenkspiegels, der ebenfalls um eine Drehachse 22 drehbar im Basisteil 20 angeordnet ist. Im vorliegenden Beispiel sind die Drehachsen des Drehgelenks 21 und des Umlenkspiegels 23 identisch und als eine gemeinsame Drehachse 22 dargestellt. Die Drehachse 22 ist orthogonal zur optischen Achse 26 des Basisteils 20 angeordnet und mit dem Basisteil 20 und dem Zwischenträger 40 verbunden.

Der Umlenkspiegel 23 des Basisteils 20 ist mit dem Drehgelenk 21 zwischen Basisteil 20 und Zwischenträger 40 gekoppelt. Dies geschieht über ein, insbesondere gabelförmiges, Koppelelement 24, das mit dem Umlenkspiegel 23 verbunden ist. Dieses Koppelelement 24 steht in Verbindung mit einem Mitnehmerelement 25, welches mit dem Basisteil 20 verbunden ist. Weiterhin steht das Koppelelement 24 mit einem Mitnehmerelement 27 in Verbindung, welches mit dem Zwischenträger 40 verbunden ist. Wird nun das Basisteil 20 oder der Zwischenträger 40 gedreht, greifen die Mitnehmerelemente 25, 27 und das Koppelelement 24 ineinander, so dass der Umlenkspiegel 23 mitgedreht wird, vorzugsweise um den halben Drehwinkel.

Weiterhin kann das Basisteil 20 in seinem Bereich, der zur Anlage an die optische Beobachtungseinrichtung dient, eine Glasplatte aufweisen, die unter anderem zum Schutz gegen Verschmutzungen dient.

Ähnlich ist der Okularträger 30 aufgebaut. Der Okularträger 30 verfügt ebenfalls über ein Umlenkelement 34 in Form eines Umlenkspiegels, der ebenfalls um eine Drehachse 33 drehbar im Okularträger 30 angeordnet ist. Die Drehachse 33 ist im vorliegenden Beispiel parallel zur Drehachse 22 angeordnet und mit dem Okularträger 30 und dem Zwischenträger 40 verbunden. Im vorliegenden Beispiel sind die Drehachsen des Drehgelenks 32 und des Umlenkspiegels 34 identisch und als eine gemeinsame Drehachse 33 dargestellt.

Der Umlenkspiegel 34 des Okularträgers 30 ist mit dem Drehgelenk 32 zwischen Okularträger 30 und Zwischenträger 40 gekoppelt. Dies geschieht ebenfalls über ein, insbesondere gabelförmiges, Koppelelement 35, das mit dem Umlenkspiegel 34 verbunden ist. Dieses Koppelelement 35 steht in Verbindung mit einem Mitnehmerelement 36, welches mit dem Okularträger 30 verbunden ist, und mit einem Mitnehmerelement 37, welches mit dem Zwischenträger 40 verbunden ist. Wird nun der Okularträger 30 oder der Zwischenträger 40 gedreht, greifen die Mitnehmerelemente 36, 37 und Koppelelement 35 ineinander, so dass der Umlenkspiegel 34 mitgedreht wird, vorzugsweise um den halben Drehwinkel.

Für jeden Einblickwinkel sind nunmehr unterschiedliche Einblickpositionen beziehungsweise Bauhöhen und Baulängen des Schwenktubus 10 realisierbar, wie in den Figuren 6 bis 11 dargestellt ist.

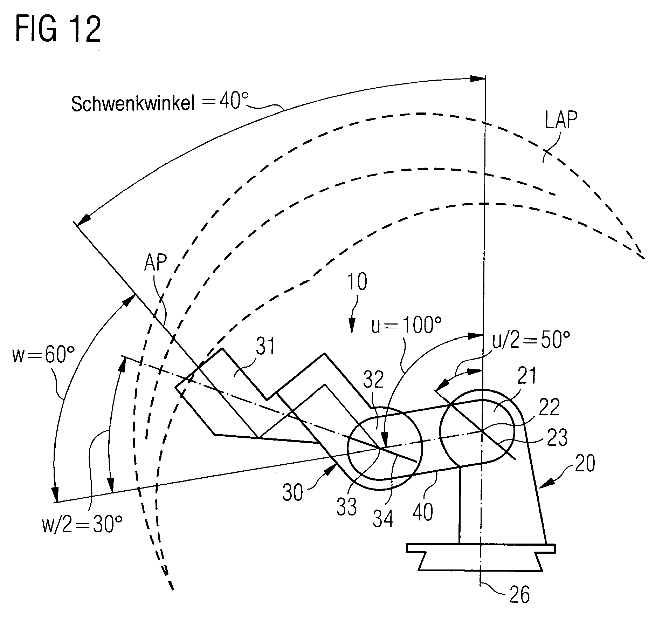

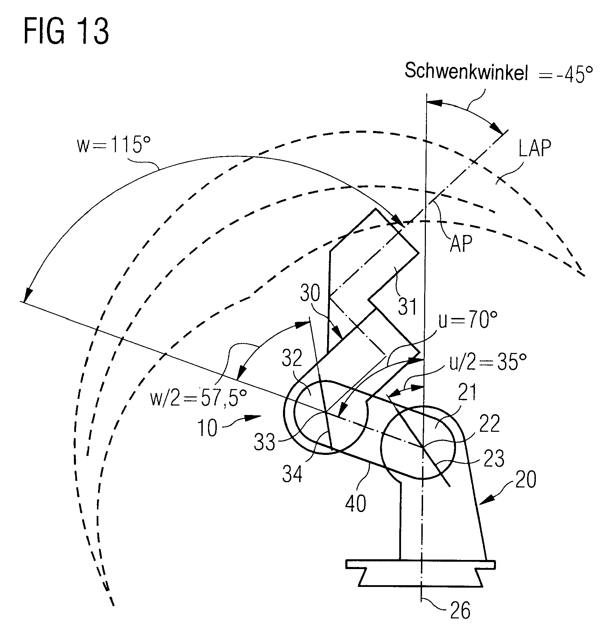

In den Figuren 12 und 13 sind verschiedene Winkelzusammenhänge beim Verschwenken eines erfindungsgemäßen Schwenktubus 10 dargestellt, der in der wie vorstehend beschriebenen Weise ausgestaltet ist.

Figur 12 zeigt einen Schwenkwinkel des Schwenktubus von 40°, während Figur 13 einen Schwenkwinkel von -45° darstellt. In den Figuren 12 und 13 ist mit "AP" die Austrittspupille bezeichnet, während mit "LAP" der Lagebereich der Austrittspupille gekennzeichnet ist. Mit "u" ist der Schwenkwinkel des Drehgelenks 21 des Basisteils 20 um die Drehachse 22 bezeichnet, während mit "u/2" der Drehwinkel des Umlenkspiegels 23 des Basisteils 20 um die Drehachse 22 bezeichnet ist. Mit "w" ist der Schwenkwinkel des Drehgelenks 32 des Okularträgers 30 um die Drehachse 33 bezeichnet, während mit "w/2" der Drehwinkel des Umlenkspiegels 34 des Okularträgers 30 um die Drehachse 33 bezeichnet ist. Es besteht folgender Zusammenhang zwischen den Schwenkwinkeln von Basisteil, Zwischenträger und Okularträger: "Schwenkwinkel Tubus = u - w".

Wie in den Figuren 12 und 13 deutlich erkennbar ist, ist eine vorteilhafte Ausgestaltung des Schwenktubus 10 dann gegeben, wenn die Umlenkspiegel 23, 34 um einen Rotationswinkel um die jeweiligen Drehachsen 22, 33 rotieren können, der der Hälfte des Rotationswinkels der entsprechenden Drehgelenke 21, 32 um die jeweiligen Drehachsen 22, 33 entspricht.

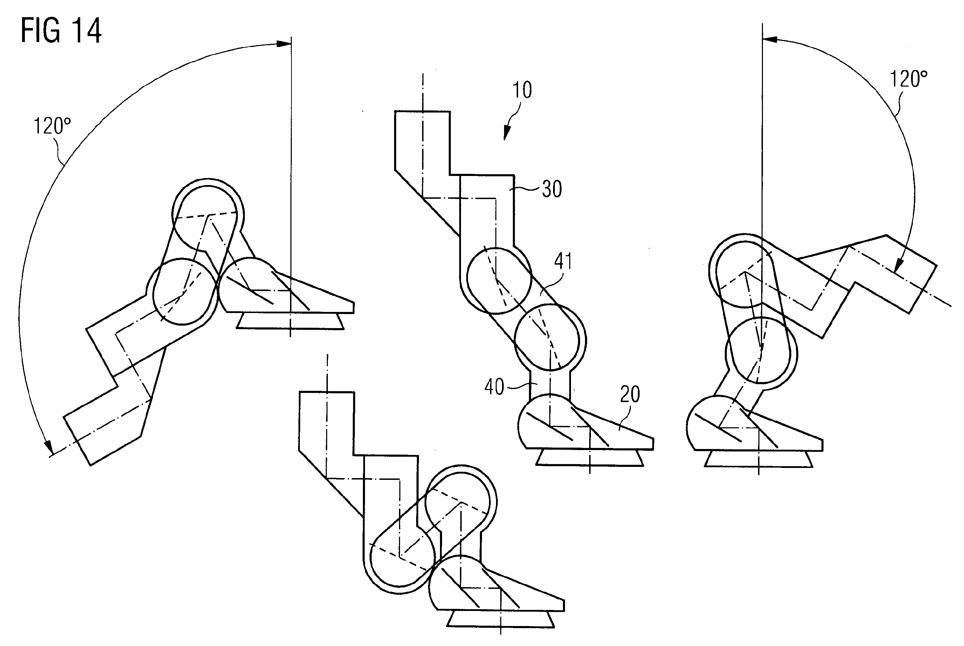

In der Figur 14 ist ein anderes Ausführungsbeispiel eines erfindungsgemäßen Schwenktubus 10 dargestellt. Der dort dargestellte Schwenktubus verfügt über drei unabhängige Gelenke. Dazu weist der Tubus 10 zunächst wiederum ein Basisteil 20 und einen Okularträger 30 auf. Zwischen diesen beiden Bauteilen befinden sich nunmehr jedoch zwei Zwischenträger 40 und 41. Der grundsätzliche Aufbau und die grundsätzliche Funktionsweise des Tubus entsprechen ansonsten den in den Figuren 6 bis 11 beschriebenen Beispielen, so dass auf die entsprechenden Ausführungen an dieser Stelle vollinhaltlich Bezug genommen und verwiesen wird. In der Figur 14 sind verschiedene Einblickpositionen für den Tubus 10 dargestellt.

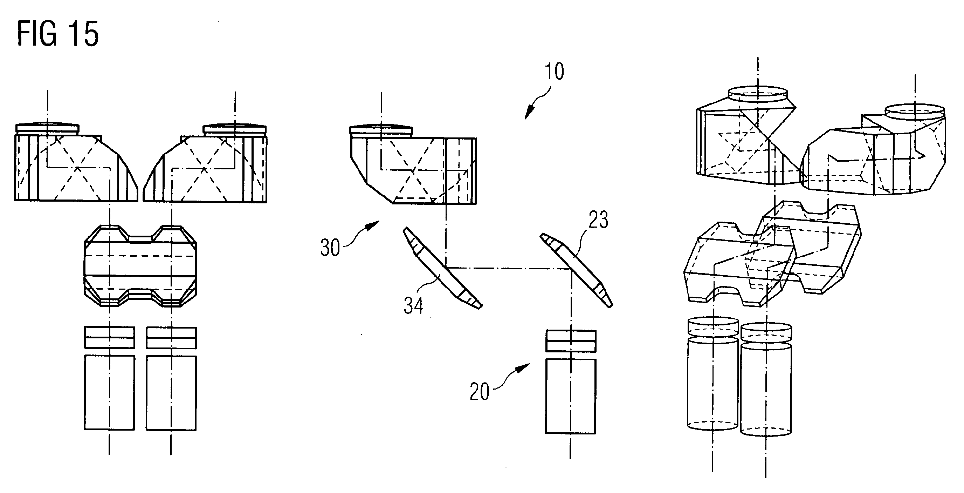

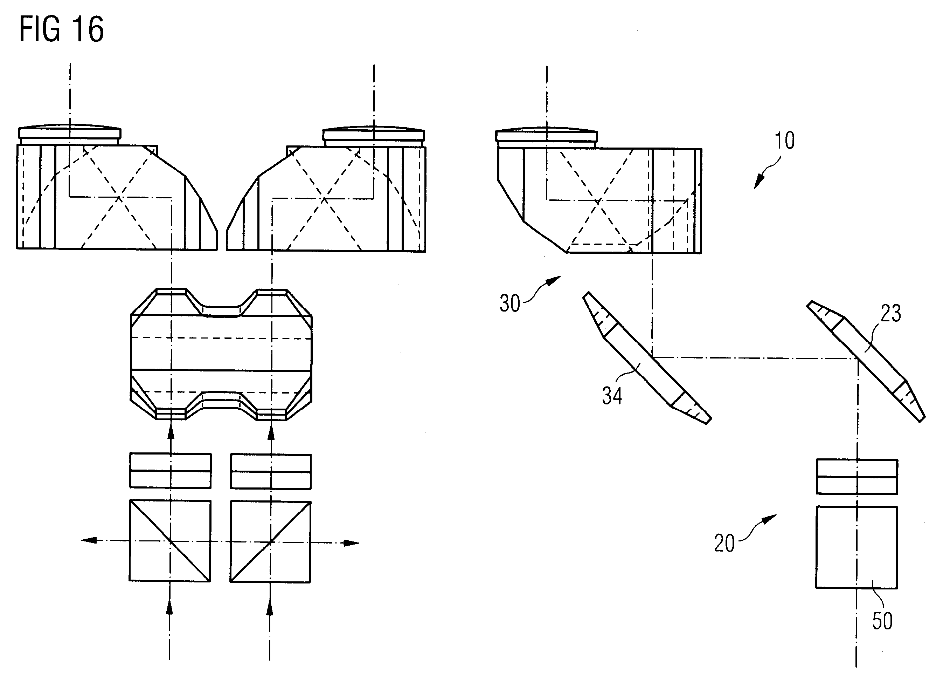

In den Figuren 15 bis 18 schließlich ist noch ein weiteres Ausführungsbeispiel für einen Schwenktubus 10 dargestellt, der von seinem Grundaufbau und seiner Grundfunktionsweise allerdings dem in den Figuren 6 bis 11 dargestellten Tubus 10 entspricht, so dass diesbezüglich auf die entsprechenden Ausführungen vollinhaltlich Bezug genommen und verwiesen wird. Der Tub8us ist als Binokulartubus ausgebildet und verfügt über zwei getrennte Strahlengänge für eine Stereomikroskopie. Der Tubus 10 verfügt wiederum über ein Basisteil 20, einen Okularträger 30 und zwei unabhängig voneinander drehbare Umlenkelemente 23 und 34. Zusätzlich verfügt der Tubus gemäß Figur 16 über ein Auskoppelelement 50. Das Auskoppelelement 50 kann anstelle der Glasplatte 28 (siehe Figuren 6 bis 11) vorgesehen sein und als Teilerelement, etwa als Teilerwürfel, Teilerplatte oder dergleichen ausgebildet sein. Es kann beispielsweise zum Auskoppeln von Strahlen für Dokumentationseinrichtungen verwendet werden. In Figur 16 befindet sich in beiden Strahlengängen des Mikroskops ein solches Auskoppelelement 50. Die Richtung der Auskopplung ist dabei durch entsprechende Pfeile im linken Teil von Figur 16 dargestellt.

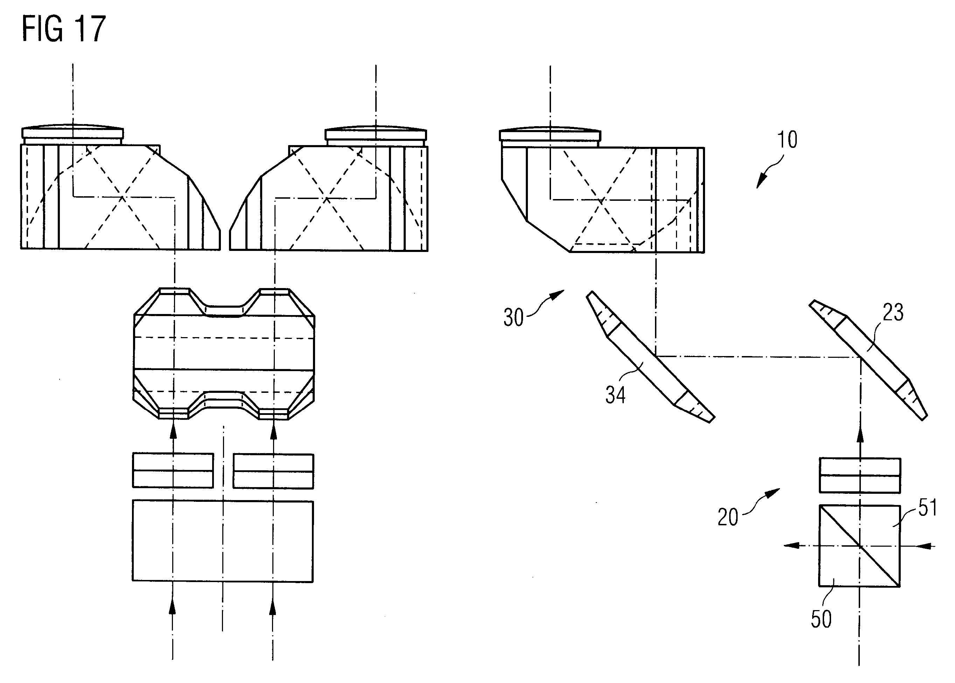

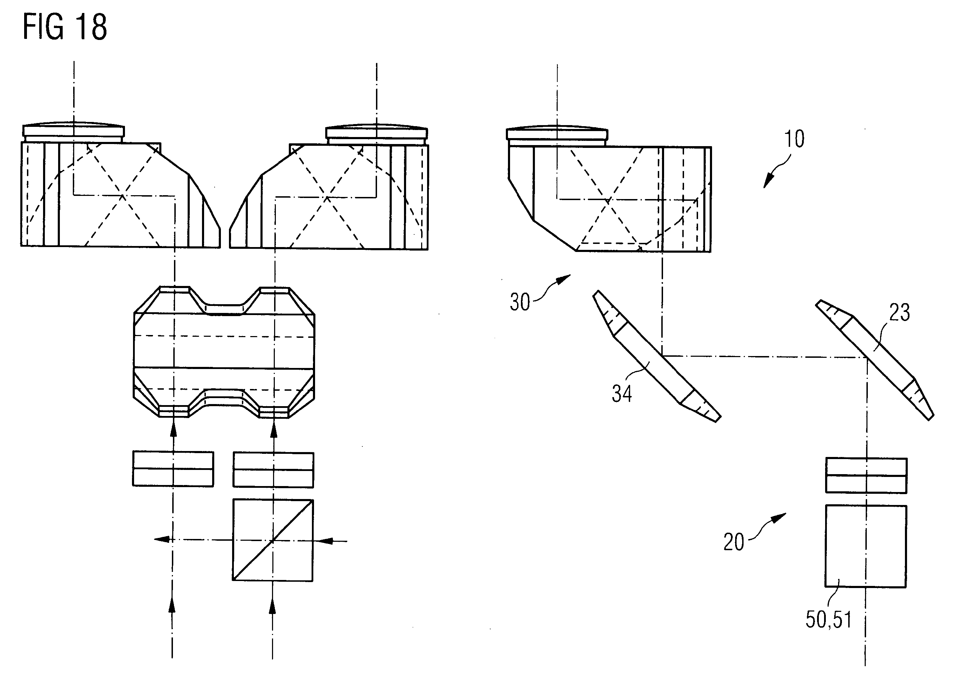

In Figur 17 ist eine Lösung dargestellt, bei der sowohl ein Auskoppelelement 50, als auch ein Einkoppelelement 51 vorgesehen ist. Das Einkoppelelement 51 kann wie das Auskoppelelement 50 ausgebildet sein (siehe oben), etwa als Teilerwürfel, Teilerspiegel oder dergleichen. Es kann dazu genutzt werden, um Informationen aus einer Dateneinspiegelungseinrichtung in den Strahlengang einzuspiegeln. Bei dem in Figur 17 dargestellten Beispiel befinden sich die Einkoppelelemente 51 und Auskoppelelemente 50 ebenfalls in beiden Strahlengängen der als Stereomikroskop ausgebildeten Beobachtungseinrichtung. Die Einkoppel.- und Auskoppelrichtung ist durch entsprechende Pfeile im rechten Teil von Figur 17 dargestellt. Das in Figur 18 dargestellte Beispiel entspricht von seinem Grundaufbau her dem in Figur 17 gezeigten Beispiel, nur dass sich die Einkoppel- und Auskoppelelemente 50, 51 bei dem in Figur 18 gezeigten Beispiel nur in einem Strahlengang befinden.

### Bezugszeichenliste

- 10: Tubus (Schwenktubus)

- 20: Basisteil
- 21: Drehgelenk
- 22: Drehachse
- 23: Optisches Element (Umlenkelement)
- 24: Koppelelement
- 25: Mitnehmerelement (Basisteil)
- 26: Mikroskopachse
- 27: Mitnehmerelement (Zwischenträger)
- 28: Glasplatte

- 30: Okularteil
- 31: Okularaufnahme
- 32: Drehgelenk
- 33: Drehachse
- 34: Optisches Element (Umlenkelement)
- 35: Koppelelement
- 36: Mitnehmerelement (Okularträger)
- 37: Mitnehmerelement (Zwischenträger)

- 40: Zwischenträger
- 41: Zwischenträger

- 50: Auskoppelelement
- 51: Einkoppelelement

- AP: Austrittspupille
- LAP: Lagebereich der Austrittspupille

## Patentansprüche

1. Tubus (10), insbesondere Schwenktubus, für eine optische Beobachtungseinrichtung, insbesondere für ein Mikroskop, mit einer in Länge und/oder Höhe linear variablen Einblickhöhe, wobei der Tubus (10) einen optischen Lichtweg aufweist, in dem wenigstens zwei optische Umlenkelemente (23, 34) unabhängig voneinander beweglich angeordnet sind, wobei der Tubus (10) ein Basisteil (20) zur Anlage an einer optischen Beobachtungseinrichtung aufweist, in dem ein um eine erste Drehachse (22) drehbares Umlenkelement (23) angeordnet ist, wobei der Tubus (10) einen zum Basisteil (20) verschwenkbaren Okularträger (30) aufweist, in dem ein um eine zweite Drehachse (33) drehbares Umlenkelement (34) angeordnet ist, wobei der Okularträger (30) über wenigstens einen Zwischenträger (40, 41) verschwenkbar am Basisteil (20) angeordnet ist, wobei der Zwischenträger (40) über ein Drehgelenk (21) mit dem Basisteil (20) und über ein Drehgelenk (32) mit dem Okularträger (30) verbunden ist, wobei das Umlenkelement (23) des Basisteils (20) mit dem Drehgelenk (21) zwischen Basisteil (20) und Zwischenträger (40) gekoppelt ist und wobei dass das Umlenkelement (34) des Okularträgers (30) mit dem Drehgelenk (32) zwischen Okularträger (30) und Zwischenträger (40) gekoppelt ist, **dadurch gekennzeichnet, dass** das Umlenkelement (23) des Basisteils (20) mit einem Koppelelement (24) verbunden ist, welches mit einem Mitnehmerelement (25), welches mit dem Basisteil (20) verbunden ist, und mit einem Mitnehmerelement (27), welches mit dem Zwischenträger (40) verbunden ist, in Verbindung steht, und dass das Umlenkelement (34) des Okularträgers (30) mit einem Koppelelement (35) verbunden ist, welches mit einem Mitnehmerelement (36), welches mit dem Okularträger (30) verbunden ist, und mit einem Mitnehmerelement (37), welches mit dem Zwischenträger (40) verbunden ist, in Verbindung steht.

2. Tubus nach Anspruch 1, **dadurch gekennzeichnet, dass** dieser derart ausgebildet ist, dass sich der optische Lichtweg des Tubus (10) bei einer Variierung der Einblickhöhe um weniger als 30% ändert.

3. Tubus nach Anspruch 1 oder 2, **dadurch gekennzeichnet, dass** dieser in seiner mechanischen Länge variabel einstellbar ausgebildet ist.

4. Tubus nach einem der Ansprüche 1 bis 3, **dadurch gekennzeichnet, dass** wenigstens ein optisches Umlenkelement (23, 34) als Umlenkspiegel ausgebildet ist.

5. Tubus nach einem der Ansprüche 1 bis 4, **dadurch gekennzeichnet, dass** die Umlenkelemente (23, 34) eine translatorische Komponente aufweisen und/oder dass die Umlenkelemente (23, 34) rotatorisch unabhängig voneinander schwenkbar angeordnet sind.

6. Tubus nach einem der Ansprüche 1 bis 5, **dadurch gekennzeichnet, dass** das Umlenkelement (23) des Basisteils (20) und das Drehgelenk (21) eine identische Drehachse (22) aufweisen und/oder dass das Umlenkelement (34) des Okularträgers (30) und das Drehgelenk (32) eine identische Drehachse (33) aufweisen.

7. Tubus nach Anspruch 6, **dadurch gekennzeichnet, dass** die Drehachse (22) des Umlenkelements (23) des Basisteils (20) parallel zur Drehachse (33) des Umlenkelements (34) des Okularträgers (30) angeordnet ist.

8. Tubus nach einem der Ansprüche 1 bis 7, **dadurch gekennzeichnet, dass** in dem Basisteil (20) wenigstens ein optisches Auskoppelelement (50) und/oder wenigstens ein optisches Einkoppelelement (51) angeordnet ist/sind.

9. Tubus nach einem der Ansprüche 1 bis 8, **dadurch gekennzeichnet, dass** der Tubus (10) für einen monokularen Einblick oder als Binokulartubus ausgebildet ist.

## Claims

1. Tube (10), more particularly swivel tube, for an optical observation device, more particularly for a microscope, comprising a viewing height which is linearly variable in terms of length and/or height, wherein the tube (10) has an optical light path in which at least two optical deflection elements (23, 34) are arranged in manner in which they can move independently of one another, wherein the tube (10) has a base part (20) for application on an optical observation device, in which a deflection element (23) rotatable about a first axis of rotation (22) is arranged, wherein the tube (10) has an eyepiece holder (30) swivelable in relation to the base part (20), in which a deflection element (34) rotatable about a second axis of rotation (33) is arranged, wherein the eyepiece holder (30) is arranged in a swivelable manner on the base part (20) by way of at least one intermediate holder (40, 41), wherein the intermediate holder (40) is connected to the base part (20) by way of a rotary joint (21) and to the eyepiece holder (30) by way of a rotary joint (32), wherein the deflection element (23) of the base part (20) is coupled to the rotary joint (21) between the base part (20) and the intermediate holder (40) and wherein the deflection element (34) of the eyepiece (30) is coupled to the rotary joint (32) between the eyepiece holder (30) and the intermediate holder (40), **characterized in that** the deflection element (23) of the base part (20) is connected to a coupling element (24) which is connected to a catch element (25) which is connected to the base part (20) and to a catch element (27) which is connected to the intermediate holder (40) and **in that** the deflection element (34) of the eyepiece holder (30) is connected to a coupling element (35) which is connected to a catch element (36) which is connected to the eyepiece holder (30) and to a catch element (37) which is connected to the intermediate holder (40).

2. Tube according to Claim 1, **characterized in that** it is embodied in such a way that the optical light path of the tube (10) changes by less than 30% when varying the viewing height.

3. Tube according to Claim 1 or 2, **characterized in that** it has a variably adjustable embodiment in terms of the mechanical length thereof.

4. Tube according to one of Claims 1 to 3, **characterized in that** at least one optical deflection element (23, 34) is embodied as a deflection mirror.

5. Tube according to one of Claims 1 to 4, **characterized in that** the deflection elements (23, 34) have a translational component and/or **in that** the deflection elements (23, 34) are swivelably arranged in manner rotationally independent of one another.

6. Tube according to one of Claims 1 to 5, **characterized in that** the deflection element (23) of the base part (20) and the rotary joint (21) have an identical axis of rotation (22) and/or **in that** the deflection element (34) of the eyepiece holder (30) and the rotary joint (32) have an identical axis of rotation (33).

7. Tube according to claim 6, **characterized in that** the axis of rotation (22) of the deflection element (23) of the base part (20) is arranged parallel to the axis of rotation (33) of the deflection element (34) of the eyepiece holder (30).

8. Tube according to one of Claims 1 to 7, **characterized in that** at least one optical output coupling element (50) and/or at least one optical input coupling element (51) is/are arranged in the base part (20).

9. Tube according to one of Claims 1 to 8, **characterized in that** the tube (10) is embodied for a monocular eyepiece or as a binocular tube.

## Revendications

1. Tube (10), notamment tube pivotant pour un système d'observation optique, notamment pour un microscope, avec une hauteur d'observation linéaire variable en longueur et/ou en hauteur, le tube (10) comportant un chemin optique dans lequel au moins deux éléments de renvoi (23, 34) optiques sont placés en étant mobiles indépendamment l'un de l'autre, le tube (10) comportant une pièce d'embase (20) destinée à être appuyée sur un système d'observation optique, dans laquelle est placé un élément de renvoi (23) rotatif autour d'un premier axe de rotation (22), le tube (10) comportant un porte-oculaire (30) susceptible de pivoter vers la pièce d'embase (20) dans lequel est placé un élément de renvoi (34) rotatif autour d'un deuxième axe de rotation (33), le porte-oculaire (30) étant placé sur la pièce d'embase (20) en étant susceptible de pivoter par l'intermédiaire d'au moins un support intermédiaire (40, 41), le support intermédiaire (40) étant relié par l'intermédiaire d'une articulation rotative (21) avec la pièce d'embase (20) et par l'intermédiaire d'une articulation rotative (32) avec le porte-oculaire (30), l'élément de renvoi (23) de la pièce d'embase (20) étant couplé avec l'articulation rotative (21) entre la pièce d'embase (20) et le support intermédiaire (40) et que l'élément de renvoi (34) du porte-oculaire (30) étant couplé avec l'articulation rotative (32) entre le porte-oculaire (30) et le support intermédiaire (40), **caractérisé en ce que** l'élément de renvoi (23) de la pièce d'embase (20) est relié avec un élément de couplage (24), lequel est en liaison avec un élément d'entraînement (25), lequel est relié avec la pièce d'embase (20) et avec un élément d'entraînement (27), lequel est relié avec le support intermédiaire (40) et **en ce que** l'élément de renvoi (34) du porte-oculaire (30) est relié avec un élément de couplage (35), lequel est en liaison avec un élément d'entraînement (36), lequel est relié au porte-oculaire (30) et avec un élément d'entraînement (37), lequel est relié avec le support intermédiaire (40).

2. Tube selon la revendication 1, **caractérisé en ce que** celui-ci est conçu de sorte que lors d'une variation de la hauteur d'observation, le chemin optique du tube (10) se modifie de moins de 30 %.

3. Tube selon la revendication 1 ou la revendication 2, **caractérisé en ce que** celui-ci est conçu de sorte à être réglable de manière variable dans sa longueur mécanique.

4. Tube selon l'une quelconque des revendications 1 à 3, **caractérisé en ce qu'**au moins un élément de renvoi (23, 34) optique est conçu en tant que miroir de déviation.

5. Tube selon l'une quelconque des revendications 1 à 4, **caractérisé en ce que** les éléments de renvoi (23, 34) comportent un composant en translation et/ou **en ce que** les éléments de renvoi (23, 34) sont placés en étant susceptibles de pivoter en rotation indépendamment les uns des autres.

6. Tube selon l'une quelconque des revendications 1 à 5, **caractérisé en ce que** l'élément de renvoi (23) de la pièce d'embase (20) et l'articulation rotative (21) présentent un axe de rotation (22) identique et/ou **en ce que** l'élément de renvoi (34) du porte-oculaire (30) et l'articulation rotative (32) présentent un axe de rotation (33) identique.

7. Tube selon la revendication 6, **caractérisé en ce que** l'axe de rotation (22) de l'élément de renvoi (23) de la pièce d'embase (20) est placé à la parallèle de l'axe de rotation (33) de l'élément de renvoi (34) du porte-oculaire (30).

8. Tube selon l'une quelconque des revendications 1 à 7, **caractérisé en ce que** dans la pièce d'embase (20) est/sont placé(s) au moins un élément de découplage (50) optique et/ou au moins un élément de couplage (51) optique.

9. Tube selon l'une quelconque des revendications 1 à 8, **caractérisé en ce que** le tube (10) est conçu pour une observation monoculaire ou en tant que tube de binoculaire.
